(19) Europäisches Patentamt
European Patent Office
Office européen des brevets

(11) Publication number: 0 009 325 A1

(12) # EUROPEAN PATENT APPLICATION

(21) Application number: 79301706.2

(22) Date of filing: 21.08.79

(51) Int. Cl.³: A 61 K 7/16

(30) Priority: 01.09.78 US 939015

(43) Date of publication of application: 02.04.80 Bulletin 80/7

(84) Designated Contracting States: BE CH DE FR GB IT LU NL SE

(71) Applicant: Pfizer Inc., 235 East 42nd Street, New York, N.Y. (US)

(72) Inventor: Beereboom, John J., 110 Library Lane, Old Lyme New London, Connecticut (US)

(74) Representative: Graham, Philip Colin Christison et al. Pfizer Limited Ramsgate Road, Sandwich, Kent CT13 9NJ (GB)

(54) **Anti-caries composition and method using erythritol as cariostatic agent.**

(57) This invention refers to an infrared light radiation communication equipment.

The equipment comprises a headset (2), which is to be carried on the head of a person (1) and which includes ear protectors (3). At least one of the ear protectors (3) in the headset (2) includes an infrared light radiation receiver (9), a loudspeaker (5), a voice microphone (4), a noise sensing microphone (7), and infrared light radiation emitting transmitters (8). The transmitters in the ear protectors (3) are of the omnidirectional type, and persons each being provided with a headset can communicate with one another provided that they are not too far apart.

EP 0 009 325 A1

TITLE MODIFIED
see front page

## Erythritol Containing Anti-Caries Compositions

Polyhydric alcohols, or polyols, are valuable aids in formulation a wide variety of food products as described in Handbook of Food Additives, Chapter 11, published by the Chemical Rubber Company, Cleveland, Ohio. They may be used to impart special effects to the texture or taste of a food product. Since they are generally hygroscopic, they may be used to hold moisture.

Many polyhydric alcohols are present in natural foods, either in the free state or chemically combined. Glycerol is by far the most plentiful polyol found in natural foods. However, it is almost exclusively present in chemical combination as fats (tri-esters of long chain fatty acids) in meats and some vegetables. Sorbitol is found uncombined in many fruits such as apples, pears and berries, and also in some other plants. Mannitol occurs at levels as high as 90% in the manna of some types of trees as well as in a variety of vegetables and fruits. Erythritol, mannitol, arabitol and glycerol are known to be produced by the propagation of a variety of yeasts in aqueous nutrient media.

Polyhydric alcohols are used in the manufacture of foods, either to promote the retention of the original quality of the food on aging and shipping to the consumer, or to gain a texture or product quality that was not present in the original formula. These qualities are achieved through physical or chemical effects in which the polyols behave as:

1. crystallization modifiers
2. hygroscopic agents
3. softening agents
4. sweetness controllers
5. solvent agents
6. viscosity agents
7. rehydration aids
8. dietary agents

Sorbitol, mannitol, glycerol and propylene glycol, a synthetic polyhydric alcohol, are presently used as food additives. Propylene glycol must be used with care because of its adverse taste.

Erythritol is about 60% as sweet as sucrose at practical use levels and is sweeter than mannitol and sorbitol which are only 50% as sweet as sucrose under similar conditions. It has an advantage over propylene glycol and glycerol in having a clean and pleasant taste and it is very soluble in water (a saturated solution contains about 38% w/w). Unlike other polyols, erythritol is not significantly metabolized after oral ingestion in humans, and so is of unique interest as a non-nutritive sweetening agent. Commercially available erythritol is prepared as a white crystalline product by treating an aqueous alkali carbonate solution of 2-buten-1,4-diol with chlorine and saponifying the resulting chloro-hydrin. The natural product can be produced on a commercial scale by propagating specially selected yeast strains in appropriate aqueous nutrient media. It is of special interest to note that erythritol can be produced much more cheaply than certain other polyols such as xylitol.

The use of certain polyols to retard the formation of dental caries has been known for some years. For

example, Muhlemann, Regolati and Marthaler in the Helv. Odont. Acta., April 1970, p. 48-51, demonstrated that xylitol was more effective than sorbitol in preventing dental caries in rats but that both compounds caused diarrhea and an increased drinking water intake. Nakinen in the J. Dent. Res., V. 51, 1972, p. 403-408 showed that the formation of enzymes responsible for dental caries formation were retarded by xylitol, sorbitol and mannitol. In the Internat. J. Vit. Nutr. Res., 43, 1973, p. 227-232, Grunberg, Beskaid and Brin showed that xylitol is appreciably more effective in reducing the incidence of dental caries than is either mannitol or sorbitol. In a series of papers entitled "Turku Sugar Studies", Scheinin, Makinen and Ylitalo, Acta. Odontologica. Scandinavica. V. 22, 1974, p.383-412, thoroughly review the subject of the use of polyols in diet to reduce dental caries in man. They conclude that xylitol shows a dramatic reduction in the incidence of dental caries.

There is also a large amount of patent literature involving the use of polyols in food products and health related products. For example, U.S. 3,278,314 describes a sweetened peanut food with long shelf life containing polyhydric alcohols. U.S. 3,352,689 claims a sugarless gum with polyols such as sorbitol and mannitol as the additive which improves taste and texture over comparable sugarless gums. U.S. 3,899,593 claims a xylitol chewing gum in which at least 50% by weight of the composition is xylitol. U.S. 3,914,434 describes non-cariogenic foods and delicacies containing xylitol as a sugar substitute. U.S. 3,970,747 describes the use of xylitol as a humectant sweetener, particularly useful in toothpaste. Finally, U.S. 4,000,320 describes chewing gum with improved storage qualities in which xylitol is contained

in amounts of less than 50% by weight.

The present invention, for the first time, describes the use of erythritol in various compositions to reduce dental caries. A method of inducing cariostasis comprising administering orally to a subject susceptible to dental caries a cariostatic amount of erythritol is claimed. Preferred method of administration is in a form selected from the group consisting of chewing gum, toothpaste, candy, ice cream, carbonated beverage, and mouthwash all members of the group being sugarless, and particularly preferred are sugarless chewing gum and toothpaste. A sugarless cariostatic composition comprising at least 5% erythritol is also claimed. The composition is particularly preferred in the form of a chewing gum or a toothpaste.

The novel cariostatic compositions of this invention will comprise at least about 5% erythritol. The compositions may contain considerably more erythritol than this, indeed they may contain as much as 99.9% erythritol.

It is well known that sugar containing substances applied to the teeth between meals are more cariogenic than those used with meals. Therefore preferred embodiments of this invention are those which are used between meals such as snack foods, chewing gum and toothpaste. Candies such as fondant, fudge and hard sugarless candies are included. Dietetic ice cream, low calorie carbonated beverages and mouthwash fall within the scope of the contemplated compositions.

It will be apparent that in the prevention of dental caries it is desirable to replace sugars such as sucrose and glucose with non-cariogenic materials and in the compositions of this invention erythritol is the material of choice. It will further be apparent that

the most important sugar to be replaced is that consumed between meals in the form of such materials as snack foods. Erythritol neither promotes the growth of cariogenic bacteria in the mouth nor is it degraded in the mouth by such bacteria into caries-causing acids. Thus erythritol may play a more or less passive role by replacing sugar in certain compositions, but it is also proper to speak of it as the "active ingredient" or "active agent" in such compositions. There will be further elucidation of these points later in the specification.

The best program for preventing caries consists of proper brushing, avoidance of cariogenic foods especially between meals, proper diet and regular dental care. The present invention is intended to be a part of such a dental care program by substituting a non-cariogentic substance, erythritol, for cariogenic sugar while maintaining the good taste and esthetic appeal of sweetness in the diet.

It will be apparent that the reduction in dental caries achieved will be proportional to the amount of sugar which is replaced in the diet by erythritol and the manner and frequency of erythritol application. Thus, if erythritol-containing toothpaste and mouthwash are used exclusively, a significant reduction in caries will be noticed. If, on the other hand, erythritol-containing gum and sweetened snack foods such as candies are consumed in addition to the use of erythritol-containing toothpaste and mouthwash, a further reduction in the number of caries will be noted.

The susceptibility of humans to caries varies greatly but in general the substitution of as little as 5 grams of erythritol for sugar, especially between

meals, will reduce the incidence of caries.

A considerable body of experimental work has been done to establish the cariostatic nature of erythritol-containing compounds and also to compare erythritol to other polyols which are known to prevent caries, such as xylitol, and to known caries producers such as sugars. This study compared cariogenicity of erythritol with some known substances on the basis of several _in vitro_ tests. The information developed by the study is summarized in Table I at the end of this discussion.

All compounds used in the test were in 0.7% concentration. The same concentrations were used to determine acid production in a salivary system. _Streptococcus mutans_ strain GS-5, a human cariogenic strain, was used in all cases except the salivary test system in which the endogenous flora was used.

Klett tubes containing a tryticase-soy-yeast-cysteine nutrient and fitted with septum caps were flushed with nitrogen gas and preincubated 30 minutes at 37°C using standard sterile techniquies, then innoculated with a 2% innoculum of a suspension of _S. mutans_ GS-5 cells in phosphate buffer. These were then incubated at 37°C. Optical density was measured by Klett readings (red filter) at half hour intervals.

Lactic acid production was measured using the Sigma Kit for determination of lactic acid. This procedure measures the amount of lactic acid by the following reaction:

$$\text{Lactic Acid} + \text{NAD} \xrightarrow{\text{LDH}} \text{puvic acid} + \text{NADH}$$

$$\text{low } A_{340} \qquad\qquad \text{high } A_{340}$$

NAD is originally present in excess and the increase absorbance of 340 mm due to NADH formation becomes a measure of lactic acid originally present.

For the salivary test system, saliva was pooled from volunteers who had fasted three hours and the pH of the pooled saliva was noted. 4.95 ml of pool saliva was aseptically transferred to a series of sterile test tubes. 7% solutions of each compound were prepared and sterilized. 0.05 ml of each was aseptically added to the test tube of saliva to give a final concentration of 0.7%. The test tubes were incubated anaerobically at 37°C for four hours. The pH of each was then measured.

The first three columns in the table refer to effects of the compounds on the growth characteristics of a cariogenic strain of the bacteria Streptococcus mutans. Reduction of total growth (lowered final Klett), reduction of growth rate (increase in doubling time) and reduction of acid produced (increase in final pH) are the effects desired. By these three measures, xylitol showed the best results, with erythritol closely following.

The next six columns refer to certain effects on the metabolism of the bacteria. Columns 4-8 represent measures of the activity of an enzyme (or group of enzymes) called dextran sucrase (DSU) which is one of the principle sources of the cariogenicity of this organism. DSU converts dietary sugar into polymers which are a major ingredient of cariogenic dental plaque. Such plaque is an accumulation of these polymers, playing an essential role in caries formation and periodontal disease. Therefore, a reduction in plaque formation by means of a reduction in DSU activity is a desired effect. In particular, a reduction in "mean specific DSU activity" - that is, amount of activity per bacterial cell - is desired since that would imply a real effect on the organisms' metabolism instead of

merely a reduction in the number of cells. For these measures, xylitol and erythritol appear superior to the other substances tested. Erythritol, however, is the best in the critical measure "DSU mean specific activity" (column 8). In column 9, lactic acid production is measured. Lactic acid, produced by cariogenic bacteria in response to ingestion of a meal, can increase acidity enough to dissolve tooth mineral and initiate caries. Therefore, reduction in lactic acid production is desirable. Erythritol was clearly superior in this measure being the only substance which reduced lactic acid relative to the "no compound" control. Finally, in the last column (10) are measures of the effect on the pH of natural saliva. In this case, the natural mixed population of bacteria is used instead of a pure culture of one strain as in the previous columns. A positive number (pH increase, corresponding to reduced acidity) is the desired result. Again, erythritol is the best substance in the test by this measure.

In summary, erythritol and xylitol were superior to the other test substances in measures of total bacterial growth, growth rate, total acidity, plaque-producing enzyme activity and lactic acid production for a pure strain of cariogenic Streptococcus mutans, and in salivary pH change for a naturally occurring mixed bacterial population in human saliva. Although xylitol appeared slightly better than erythritol in *in vitro* measures of simple bacterial growth, erythritol appeared superior to xylitol in the more critical measures of bacterial production of cariogenic substances and in mixed bacterial populations in human saliva more closely approximating a real life situation.

The examples to follow are merely illustrative and in no way are intended to limit the scope of the appended claims.

# TABLE 1

| Compound | Final Klett | Doubling Time (Hrs) | Final pH | Total | DSU/L Insoluble | Soluble | % Insoluble | DSU Mean Specific Activity | Lactic Acid | Salivary pH |
|---|---|---|---|---|---|---|---|---|---|---|
| None | 195 | 0.6 | 4.80 | 1460 | 770 | 690 | 53 | 7.49 | 3.5 | 0 |
| Glucose | 243 | 0.7 | 4.43 | 945 | 525 | 420 | 55.5 | 3.91 | 7.2 | -1.4 |
| Xylitol | 153 | 0.95 | 4.95 | 515 | 285 | 330 | 46.5 | 4.06 | 3.9 | +0.05 |
| Sorbitol | 187 | 0.9 | 4.71 | 787 | 420 | 367 | 54 | 4.21 | 3.9 | -0.1 |
| Dextran T-10 | 195 | 0.8 | 4.72 | 1080 | 450 | 630 | 42 | 5.54 | 4.2 | 0 |
| Polydex. | 203 | 0.8 | 4.71 | 1005 | 525 | 480 | 52.5 | 4.96 | 5.1 | -0.25 |
| Erythritol | 190 | 0.9 | 4.80 | 665 | 355 | 310 | 53 | 3.50 | 3.2 | +0.1 |
| Maltitol | 193 | 0.7 | 4.79 | 1060 | 660 | 400 | 62.5 | 5.49 | 4.8 | 0. |

0009325

## Example I

Toothpaste:

A toothpaste formulation was prepared employing erythritol and glycerol in the following proportions:

| Ingredients: | % by Weight |
|---|---|
| Calcium Carbonate | 27.18 |
| Calcium Phosphate Dibasic | 9.03 |
| Sodium Fluoride | 0.08 |
| Erythritol 38% aqueous solution | 56.00 |
| Glycerol | 5.00 |
| Gum Tragacanth | 1.00 |
| Sodium Benzoate | 0.01 |
| Sodium Lauryl Sulfate | 1.00 |
| Spearmint Flavor | 0.60 |
| Sodium Saccharin | 0.10 |
| Total | 100.00 |

Preparation:

Dissolve sodium benzoate in erythritol solution. Add gum tragacanth and dissolve. Subsequently add glycerol and flavor and stir well. A thick solution should be attained. Meanwhile, mill (20μ), sift and premix calcium carbonate, calcium phosphate, sodium fluoride and sodium lauryl sulfate. Add at intervals with good stirring to aforementioned solution. After addition is completed continue mixing for an additional 10-15 minutes. If so desired, pass paste through a colloid mill to assure a properly homogeneous white paste mixture. The resultant product exhibited the texture of a conventional toothpaste. Moreover, it showed resistance to dryness when exposed to ambient room temperature (25°C) for a few days.

Use:

The toothpaste above is used for brushing by the conventional dentist-approved method, generally twice a day, and a significant reduction in the occurance of caries is noted over subjects using sugar-containing toothpaste.

## Example II

Fondant:

| Ingredients: | % |
|---|---|
| Saccharin | 0.1 |
| Erythritol 38% solution | 99.9 |

Preparation:

1. Mix all ingredients
2. Boil to 118°C
3. Pour on to a water jacketed fondant mill.
4. Allow to cool quiescently to 38°C, then agitate
5. Agitate to a proper fondant consistency (15-18 minutes)

## Example III

Bar Fudge:

| Ingredients: | % |
|---|---|
| Saccharin | 0.80 |
| Whole Milk | 16.60 |
| Hard Fat | 3.57 |
| Erythritol 38% solution | 47.02 |
| Salt | 1.00 |
| Fondant Example II | 23.00 |
| Water | 3.49 |
| Flavor qs to | 100 |

Preparation:

1. Cook erythritol solution and milk in a steam-jacketed kettle to 118°C
2. Cool to 94°C. Add fondant, fat and flavor, and mix well
3. Cast into foil pans

## Example IV

Cast Marshmallows:

| Ingredients: | % |
|---|---|
| Saccharin | 0.80 |
| Gelatin 225 Bloom | 4.30 |
| Fondant Example II | 10.93 |
| Erythritol 38% aqueous solution | 67.96 |

0009325

| | |
|---|---|
| Water | 15.94 |
| Color and Flavorings qs to | 100.00 |

Preparation:

1. Dissolve saccharin in erythritol solution by heating to 82°C
2. Agitate at low speed on a rotary mixer
3. Cool to 68°C and incorporate fondant
4. Slowly add gelatin in water while agitating solution at high speed
5. Whip batch to a density of four pounds per gallon
6. Add flavor and color
7. Cast into starch at 46 to 49 °C

Example V

Sugarless Hard Candy:

| Ingredients: | Parts |
|---|---|
| Erythritol 38% aqueous solution | 300.00 |
| Flavor | 0.10 |
| Color 10% aqueous solution | 0.10 |

Preparation:

1. Mix ingredients.
2. Heat to 250°C
3. Cool to 130°C, stir in color and flavor
4. Cool and allow candy to set (10-14 hours)

Use:

The hard candy above in pieces weighing about 7 grams, is consumed between meals by allowing to dissolve slowly in the mouth in the normal manner. About 2-5 pieces are eaten per day and the occurance of carries is reduced from subjects eating sugar-containing hard candy.

Example VI

Sugarless Mints:

| Ingredients: | % |
|---|---|
| Erythritol Powder | 96 |
| Magnesium stearate | q.s. |
| Flavor | q.s. to 100% |

Preparation:

1. Granulate erythritol and screen to particle size with good flow properties
2. Dry blend in magnesium stearate and compress on suitable tablet machine to desired size and hardness

Example VII

Dietetic Ice Cream:

| Ingredients: | % |
|---|---|
| Whey protein | 6.00 |
| Butterfat | 10.00 |
| Gelatin 225 Bloom | 0.25 |
| Atmos 150 Emulsifier | 0.25 |
| Erythritol Powder | 14.5 |
| Sodium saccharin | 0.011 |
| Flavor | q.s. |
| Water | qs to 100% |

Preparation:

1. Prepare with normal ice cream procedures and equipment

Example VIII

Sugarless Chewing Gum:

| Ingredients: | % |
|---|---|
| Gum Base | 27.00 |
| Erythritol Powder | 60.00 |
| Glycerol | 7.5 |
| Sodium saccharin | 0.12 |
| Flavor | 0.68 |
| Water | 4.70 |
| Total | 100 |

Preparation:

1. Feed gum base into a horizontal blade mixer and heat with steam for five minutes at 60°C.
2. When base has softened, blend in water and glycerol
3. Turn on mixer and steam
4. Slowly add erythritol
5. Add flavor

6. Remove gum from mixer and roll on gum roller to 0.075 inch thickness
7. Dust with erythritol powder
8. Wrap as desired

Use:

The above gum in sticks weighing about three grams is chewed at the rate of from 3-10 sticks per day, about 5 sticks being an average number. The sticks of gum are chewed at a more or less even rate throughout the day. A reduction in the occurance of caries will be noted over subjects chewing a similar amount of sugar-containing gum.

Example IX

Low-Calorie Carbonated Beverage:

Ingredients:

| | |
|---|---|
| Terpeneless lemon oil | 1 fl. oz. |
| Potable alcohol | 45 fl. oz. |
| Citric acid | 1.5 fl oz. |
| Sodium saccharin | 1.0 oz. |
| Water | qs to 1 gallon |

Preparation:

1. Mix oil, alcohol and 42 fluid ounces of water
2. Add saccharin and citric acid
3. Add sufficient water to make one gallon of flavor concentrate
4. Dilute 375 ml of flavor concentrate with a 38% w/w aqueous solution of erythritol sufficient to make one gallon.
5. Add one fluid ounce of diluted flavor solution per 7 ounce bottle filled with carbonated water. The final concentration of erythritol is approximately 5% w/w.

Example X

Mouthwash:

A mouthwash formulation employing erythritol for its sweetness and anti-caries properties was prepared as follows.

| Ingredients: | % by weight |
|---|---|
| Crystalline Erythritol | 10.00 |
| FD&C Yellow #5 | 0.03 |
| Sodium Borate | 0.03 |
| Water | 50.07 |
| Eucalyptol | 0.15 |
| Menthol | 0.35 |
| Anethol | 0.12 |
| Thymol | 0.10 |
| Cetylpyridinium Chloride | 0.05 |
| Benzoic Acid | 0.10 |
| Ethyl Alcohol | 39.00 |
| Total | 100.00 |

Method:

Dissolve erythritol, yellow #5 and sodium borate in water (I). In a separate beaker dissolve eucalyptol, menthol, anethol, thymol, cetylpyridinium chloride and benzoic acid in alcohol. Combine with (I).

## Claims

1. A method of inducing cariostasis comprising administering orally to a subject susceptible to dental caries a cariostatic amount of erythritol.

2. The method of Claim 1 wherein said cariostatic amount of erythritol is administered in the form of chewing gum, toothpaste, candy, ice cream, a carbonated beverage, or a mouthwash; all being sugarless.

3. The method of Claim 1 wherein said cariostatic amount of erythritol is administered in the form of sugarless chewing gum.

4. The method of Claim 1 wherein said cariostatic amount of erythritol is administered in the form of sugarless toothpaste.

5. A sugarless cariostatic composition comprising at least 5% erythritol.

6. A sugarless cariostatic composition as claimed in claim 5 in the form of chewing gum, toothpaste, candy, ice cream, a carbonated beverage or a mouthwash.

7. A sugarless cariostatic toothpaste comprising at least 5% erythritol.

8. A sugarless cariostatic chewing gum comprising at least 5% erythritol.

9. Erythritol and sugarless compositions thereof for use in the prevention or reduction of dental caries.

European Patent Office

# EUROPEAN SEARCH REPORT

Application number
EP 79 30 1706

## DOCUMENTS CONSIDERED TO BE RELEVANT

| Category | Citation of document with indication, where appropriate, of relevant passages | Relevant to claim | CLASSIFICATION OF THE APPLICATION (Int. Cl.) |
|---|---|---|---|
| D | US - A - 3 278 314 (E.E. COLBY)<br>* The claims; column 2, lines 66-71 *<br>-- | 1,5,6 | A 61 K 7/16 |
| A | FR - A - 899 573 (SOCIETE DES USINES CHIMIQUES RHONE-POULENC)<br>* The abstract; page 2, column 1, line 12 to page 2, column 2, line 4 *<br>-- | 1 | |
| A | AU - B - 492 521 (PORT DENTAL LAB.)<br>* The claims *<br>-- | 1 | TECHNICAL FIELDS SEARCHED (Int.Cl.)<br>A 61 K 7/16 |
| DA | US - A - 3 914 434 (E. BOHNI)<br>* The claims *<br>-- | 1 | |
| DA | US - A - 3 970 747 (J.B. BARTH)<br>* The claims; the abstract *<br>---- | 1 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 20-12-1979 | VANHECKE |